(19) Europäisches Patentamt
European Patent Office
Office européen des brevets

(11) Veröffentlichungsnummer: **0 062 704**
**A1**

(12) **EUROPÄISCHE PATENTANMELDUNG**

(21) Anmeldenummer: 81109868.0

(22) Anmeldetag: 25.11.81

(51) Int. Cl.³: A 61 K 31/275
A 61 K 31/215

(30) Priorität: 03.12.80 US 212419

(43) Veröffentlichungstag der Anmeldung: 20.10.82 Patentblatt 82/42

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt/Main 80(DE)

(72) Erfinder: Bonin, Werner, Dr.
Im Schulzehnten 18
D-6233 Kelkheim (Taunus)(DE)

(54) **Cyclopropancarbonsäureester-derivate als Mittel zum Bekämpfen von Magendasseln bei Equiden.**

(57) Es werden Mittel gegen Magendasseln bei Equiden, mit einem Gehalt an einem Cyclopropancarbonsäureester der Formel I

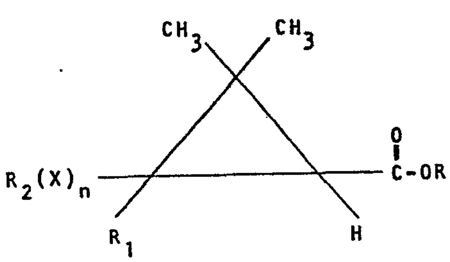

wobei
- $R_1$ Wasserstoff oder Methyl,
- $R_2$ -Alkyl, -Halogenalkyl mit 1-4 Halogenatomen wie Fluor, Chlor oder Brom, -Alkenyl, -Halogenalkenyl mit 1-4 Halogenatomen wie Fluor, Chlor oder Brom, Phenyl, Halogenphenyl mit 1-3 Halogenatomen wie Fluor oder Chlor,
- X Sauerstoff oder Schwefel und
- n 0 oder 1 bedeuten, mit der Maßgabe daß wenn
- n 0 bedeutet,

$R_1$ und $R_2$ zusammen mit dem C-Atom, an das sie gebunden sind, eine cyclische Gruppe der Formel II oder III bedeuten,

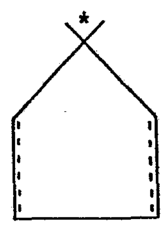

II

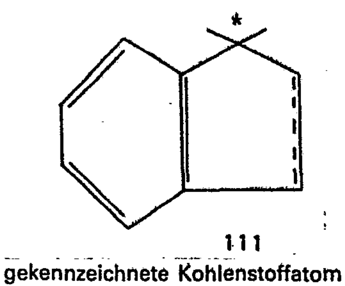

III

worin das mit einem (*) gekennzeichnete Kohlenstoffatom das mit dem Cyclopropanring gemeinsame Kohlenstoffatom ist, während ═══ eine Einfach- oder Doppelbindung darstellt.
und wobei in der Formel I R einen Rest der Formel IV, V oder VI bedeutet,

./...

EP 0 062 704 A1

Croydon Printing Company Ltd.

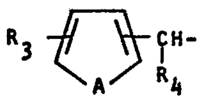 ; 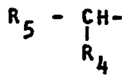 ; 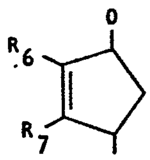

IV V VI

in welchen

- $R_4$ Wasserstoff, Äthinyl oder Cyan,
- $R_3$ Allyl, Propargyl, Benzyl, Thenyl, Furylmethyl, Phenoxy oder Phenylmercapto, welche Reste gegebenenfalls durch ein oder zwei Fluor- oder Chloratome oder durch Alkyl oder Alkoxy mit jeweils 1 bis 3 C-Atomen oder durch Trifluormethyl substituiert sein können,
- A Sauerstoff, Schwefel oder –CH=CH–,
- $R_5$ einen Phthalimid-, Thiophthalimid-, Di- oder Tetra- hydrophthalimid- oder Dialkylmaleinimidrest,
- $R_6$ ($C_1$-$C_3$)-Alkyl, ($C_2$ -$C_4$)-Alkenyl, ($C_2$-$C_4$) Alkinyl oder Benzyl und
- $R_7$ Wasserstoff oder Methyl bedeuten,

in Mischung mit einem pharmazeutisch üblichen Trägerstoff und/oder Konstituenz, beschrieben.

Mittel zur Bekämpfung von Magendasseln bei Equiden und ihre Verwendung

BEZEICHNUNG GEÄNDERT siehe Titelseite

Als Magendasseln werden die Larven der Magenbremsen bezeichnet. Magenbremsen sind Fliegen, die mit den Arten Gasterophilus intestinalis, Gasterophilus haemorrhoidalis, Gasterophilus inermis, Gasterophilus nasalis, Gasterophilus pecorum und Gasterophilus nigricornis die Gattung Gasterophilinae bilden, die der Familie der Gasterophilidae angehört. Gemeinsam ist diesen Fliegen, daß ihre Larven im Magen von Pferden, Eseln, Maultieren und Zebras eine obligat parasitische Lebensweise führen.

Sie gelangen in den Magen, indem Fliegenweibchen im Flug ihre Eier an Haare der Wirtstiere ablegen. Aus den Eiern schlüpfen die Larven I. Sie werden von den Wirtstieren abgeleckt und gelangen in den Mund. Dort bohren sie sich in die Schleimhaut des Mundes und der Zunge ein. Nach einer Wanderung zum Schlundkopf kehren sie wieder auf die Oberfläche zurück und werden von dort in den Magen abgeschluckt. Dort heften sich die Larven mittels Mundhaken in der Schleimhaut der Magenwand an. Im Magen häuten sich die Magendasseln zweimal, woraus die Larven II und Larven III hervorgehen. Die Verweildauer der Magendasseln im Magen beträgt etwa 10 Monate, danach gehen sie über den Darmtrakt ins Freie ab, wo sie sich verpuppen. Aus den Puppen geht eine neue Fliegengeneration hervor.

Während der Verweilzeit im Magen entfalten die Magendasseln als Parasiten ihre Schadwirkung. Infolge der chronischen Magenschleimhautentzündung zeigen befallene Wirtstiere je nach Schwere des Befalls Symptome von Leistungsminderung, Anaemie und Kachexie.

Deshalb ist zur Gesunderhaltung der Tiere, insbesondere von Arbeits- und Sportpferden, die Bekämpfung der

Dassellarven dringend erforderlich. Ziel der Bekämpfung ist die Abtötung und Abtreibung der Dassellarven. Dazu wurden in früheren Zeiten Schwefelkohlenstoff und gegenwärtig Phosphorsäureester, wie z.B. Dimethyl-2,2,2-trichloro-1-hydroxyäthylphosphonat (Trichlorfon), verwendet. Schwefelkohlenstoff ist lebertoxisch. Für Trichlorfon wird eine Dosierung von 35 mg/kg Körpergewicht empfohlen. Nach Anwendung dieser Dosierung werden häufig Nebenwirkungen beobachtet, die, wie von Phosphorsäureestern bekannt, auf die Hemmung der Cholinesterase zurückzuführen sind.

Es besteht deshalb ein dringender Bedarf an neuen, sicher wirksamen und gut verträglichen Tierarzneimitteln zur Bekämpfung der Magendasseln.

Überraschenderweise wurde nun gefunden, daß durch die erfindungsgemäße Verwendung von Cyclopropancarbonsäure - estern eine erfolgreiche Bekämpfung der Dassellarven möglich ist.

Dazu werden Cyclopropancarbonsäureester der allgemeinen Formel I an befallene Tiere verabreicht

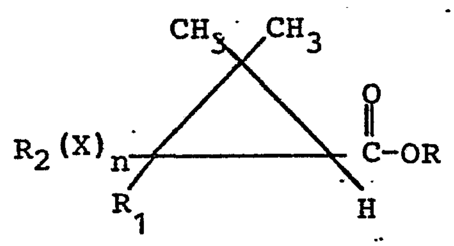

wobei

$R_1$ Wasserstoff oder Methyl,

$R_2$ $(C_1-C_4)$-Alkyl, $(C_1-C_4)$-Halogenalkyl mit 1-4 Halogenatomen wie Fluor, Chlor oder Brom, $(C_2-C_4)$-Alkenyl, $(C_2-C_4)$-Halogenalkenyl mit 1-4 Halogenatomen wie Fluor, Chlor oder Brom, Phenyl, Halogenphenyl mit 1-3 Halogenatomen wie Fluor oder Chlor,

X Sauerstoff oder Schwefel und

n 0 oder 1 bedeuten, mit der Maßgabe daß, wenn

n 0 bedeutet,
$R_1$ und $R_2$ zusammen mit dem C-Atom, an das sie gebunden sind, eine cyclische Gruppe der Formel II oder III bedeuten,

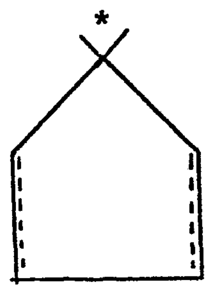

II

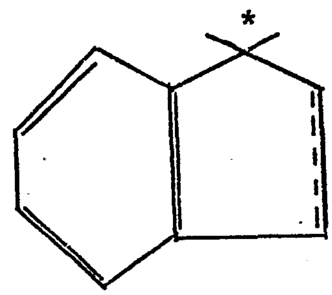

III

worin das mit einem (*) gekennzeichnete Kohlenstoffatom das mit dem Cyclopropanring gemeinsame Kohlenstoffatom ist, während ------- eine Einfach- oder Doppelbindung darstellt,

und wobei in der allgemeinen Formel I R einen Rest der Formel IV, V oder VI bedeutet,

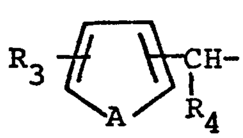 ; 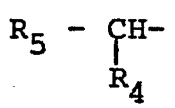 ; 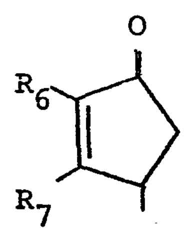

IV V VI

in welchen
$R_4$ Wasserstoff, Äthinyl oder Cyan,
$R_3$ Allyl, Propargyl, Benzyl, Thenyl, Furylmethyl, Phenoxy oder Phenylmercapto, welche Reste gegebenenfalls durch ein oder zwei Fluor- oder Chloratome oder durch Alkyl oder Alkoxy mit jeweils 1 bis 3 C-Atomen oder durch Tifluormethyl substituiert sein können,

A Sauerstoff, Schwefel oder -CH=CH-,

$R_5$ einen Phthalimid-, Thiophthalimid-, Di- oder Tetrahydrophthalimid- oder Dialkylmaleinimidrest,

$R_6$ $(C_1-C_3)$-Alkyl, $(C_2-C_4)$-Alkenyl, $(C_2-C_4)$-Alkinyl oder Benzyl und

$R_7$ Wasserstoff oder Methyl bedeuten.

Die Verbindungen der Formel I umfassen sämtliche Isomeren.

Verbindungen der Formel I sind nach Chemical Society Reviews 7, (1978) S. 473-505 als Schädlingsbekämpfungsmittel bekannt. Eine Wirksamkeit gegen Magendasseln wurde jedoch nicht beschrieben.

Bevorzugt sind zur Bekämpfung von Magendasseln gemäß der Erfindung solche Cyclopropancarbonsäureester der Formel I wobei

$R_1$ Wasserstoff

$R_2$ Halogenäthyl mit 4 Halogenatomen aus der Gruppe Chlor und Brom, ω-Dichlorvinyl oder ω-Dibromvinyl,

n Null und

R den Rest

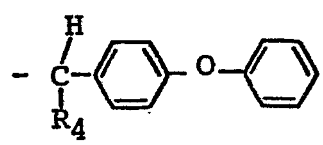

in welchem $R_4$ für Wasserstoff oder die Cyanogruppe steht, bedeuten.

Besonders bevorzugt sind (S)- α-Cyan-3-phenoxybenzyl(1R)-cis-3-(2,2-dibromvinyl)-2,2-dimethylcyclopropancarboxylat, (S)-α-Cyan-3-phenoxybenzyl(1R)-cis-3-(2,2-dichlorvinyl)-2,2-dimethyl-cyclopropancarboxylat, (S)-α-Cyan-3-phenoxybenzyl (1R)-cis-3-(2',2'-dichlor-1', 2'-dibromäthyl)-2,2-dimethylcyclopropancarboxylat, α-Cyan-3-phenoxybenzyl-3-(2,2-dichlorvinyl)-2,2-dimethylcyclopropancarboxylat, 3-Phenoxybenzyl-3(2,2-dichlorvinyl)-2,2-dimethylcyclopropancarboxylat.

Die erfindungsgemäß zu verwendenden Verbindungen der Formel I werden in Dosierungen von 0,1 - 100 mg/kg Körpergewicht, bevorzugt 0,5 - 50 mg/kg Körpergewicht, zweckmäßig auf oralem Wege verabreicht. Dabei werden überraschenderweise die erfindungsgemäß zu verwendenden Verbindungen der Formel I durch Enzyme des Speichels und des Magens nicht gespalten, sondern sie entfalten im Magen ihre volle Wirkung. Dadurch werden die Magendasseln abgetötet und durch den Verdauungskanal abgetrieben.

Zur Verabreichung an Equiden können die Wirkstoffe nach Formel I mit den üblichen inerten, flüssigen oder festen Trägerstoffen in bekannte Arzneizubereitungen, wie Pasten, Pulver, Tabletten, Boli, Suspensionen, Emulsionen, Granulaten und Lösungen eingearbeitet werden. Sie können auch zu Arzneizubereitungen verarbeitet werden, die außerdem Anthelmintika enthalten, so daß eine gleichzeitige Bekämpfung von Magen-Darm-Würmern, Saugwürmern, Bandwürmern und Magendasseln möglich ist. In diesen Arzneizubereitungen sind die Verbindungen der Formel I zu 0,01 bis 50 Gewichts-%, bevorzugt zu 0,2 - 20 Gewichtsprozent enthalten.

Die Erfindung wird durch die folgenden Beispiele erläutert.

Beispiel 1: Beispiel für eine Arzneizubereitung

a) 100 g einer flüssigen Arzneizubereitung setzen sich wie folgt zusammen:

| | |
|---|---|
| Verbindung gemäß Formel I (Verbindung A,B oder C nach Beispiel 2) | 5,00 g |
| Phenylsulfonat | 19,00 g |
| Rizinusöl, äthoxyliert (Emulsogen (R) EL 360) | 6,00 g |
| Capryl/Caprinsäure-1,2-propandiol-diester | 70,00 g |

Diese pharmazeutische Zubereitung kann zur Dassellarvenbehandlung den Equiden unverdünnt oder mit Wasser verdünnt eingegeben werden.

b)

| | |
|---|---|
| Verbindungen gemäß Formel I (Verbindung A,B oder C nach Beispiel 2) | 1 - 10 Gew.-% |
| Lactose | 10 - 30 Gew.-% |
| Maisstärke | 10 - 20 Gew.-% |
| Siliciumdioxid | 1 - 10 Gew.-% |
| Natrosol (R) JR 250 | 1 - 5 Gew.-% |
| Talkum | 1 - 5 Gew.-% |
| Ultraamylopektin | zu 100 Gew.-% |

Zur Herstellung von Boli von je 5 g Gewicht zur oralen Applikation.

c)

| | |
|---|---|
| Verbindung gemäß Formel I (Verbindung A,B oder C nach Beispiel 2) | 1 - 10 Gew.-% |
| p-Hydroxybenzosäuremethylester | 1 - 10 Gew.-% |
| Propylenglykol | 10 - 40 Gew.-% |
| Glycerin | 10 - 30 Gew.-% |
| Wasser | zu 100 Gew.-% |

Zur Herstellung einer Paste zur oralen Applikation.

d) Verbindung gemäß Formel I (Verbindung A,B oder C nach Beispiel 2 | 1 - 10 Gew.-%

| | |
|---|---|
| d) Verbindung gemäß Formel I (Verbindung A,B oder C nach Beispiel 2 | 1 - 10 Gew.-% |
| Siliciumdioxid | 10 - 30 Gew.-% |
| Calciumcarbonat | zu 100 Gew.-% |

Zur Herstellung eines Pulvers zur oralen Applikation.

Beispiel 2 : In vitro Test an Larven von Gasterophilus intestinalis

Die Verbindungen (S)-α-Cyan-3-phenoxybenzyl(1R)-cis-3-(2,2-dibromvinyl)-2,2-dimethylcyclopropancarboxylat, nachfolgend bezeichnet als Verbindung A, (S)-α-Cyan-3-phenoxybenzyl(1R)-cis-3-(2,2-dichlorvinyl)-2,2-dimethylcyclopropancarboxylat, nachfolgend bezeichnet als Verbindung B, (S)-α-Cyan-3-phenoxybenzyl(1R)-cis-3-(2',2'-dichlor-1',2'-dibromäthyl)-2,2-dimethylcyclopropancarboxylat, nachfolgend bezeichnet als Verbindung C, werden in Zubereitungen nach Beispiel 1 a) so in Wasser verdünnt, daß die Verdünnungen 0,1 % der Wirkstoffe enthalten. Zum Vergleich wird Dimethyl-2,2,2-trichloro-1-hydroxyäthyl-phosphonat (Trichlorfon) in derselben Verdünnung verwendet.

Larven III der Magenbremse, Gasterophilus intestinalis, werden aus dem Magen eines geschlachteten Pferdes entnommen. Jeweils zehn Larven werden in Bechergläsern in die Verdünnungen für 5 Minuten eingetaucht. Zur Kontrolle werden 10 Larven in Wasser eingetaucht. Danach werden die Larven auf Filterpapier getrocknet und in Behälter aus Styropor verbracht, die unter einer Wärmelampe bei etwa 37 °C aufbewahrt werden. Nach 1, 6, 24, 48, 72, 96 und 120 Stunden werden alle Larven untersucht und deren Mortalität protokolliert.

Aus der nachfolgenden Tabelle 1 geht die überlegene Wirksamkeit der Verbindungen A, B und C gegenüber dem

Vergleichspräparat Trichlorfon hervor.

Tabelle 1:

| | Zahl toter Larven | Stunden nach Behandlung | | | | | |
|---|---|---|---|---|---|---|---|
| Verbindung | 1 | 6 | 24 | 48 | 72 | 96 | 120 |
| A | 10 | 10 | 10 | 10 | 10 | 10 | 10 |
| B | 10 | 10 | 10 | 10 | 10 | 10 | 10 |
| C | 4 | 10 | 10 | 10 | 10 | 10 | 10 |
| Trichlorfon | 6* | 7* | 4* | 5* | 9* | 6* | 6* |
| Kontrolle | 0 | 0 | 0 | 1 | 2 | 2 | 2 |

* Larven erholen sich zum Teil wieder, so daß deren Mortalität nicht eindeutig zu bestimmen ist.

Beispiel 3: In vivo-Prüfung an Pferden mit Magendasselbefall

Zwei Pferde mit Körpergewichten von 190 kg und 142 kg werden mit den Verbindungen A bzw. C aus Beispiel II behandelt. Die Dosis beträgt 10 mg/kg Körpergewicht. Die sich aus der Dosis und der Konzentration der Arzneizubereitung nach Beispiel 2 zu errechnende Menge der Zubereitung wird im Volumenverhältnis 1 : 1 mit Wasser verdünnt und den Pferden mittels Nasenschlundsonde verabreicht.

Nach der Behandlung werden die Pferde in Einzelboxen aufgestellt. Der Kot wird für 5 Tage gesammelt und auf das Vorhandensein von abgegangenen Dassellarven untersucht. Vorhandene Dassellarven werden gezählt und ihre Artzugehörigkeit bestimmt. Am siebten Tag nach der Behandlung werden die Pferde getötet und der Magen-Darmtrakt auf das Vorhandensein noch angehefteter und toter Dassellarven untersucht.

Die Zahl der abgegangenen und toten Larven wird auf die Gesamtzahl vorhandener Larven prozentual bezogen und ihr Prozentsatz als "% Wirksamkeit" bezeichnet. Somit besagen 100 %, daß alle und 0 %, daß keine Larven abgetötet und abgetrieben werden.

Die nachfolgende Tabelle 2 zeigt, daß in diesen Versuchen die Verbindung A gegenüber Gasterophilus intestinalis 93 % und gegenüber Gasterophilus nasalis 100 % Wirksamkeit und daß Verbindung C gegenüber beiden Arten 100 % Wirksamkeit erbrachte.

**Tabelle 2 :**

| Dassellarven | Zahl der Larven im Kot an den Tagen nach der Behandlung 1 | 2 | 3 | 4 | 5 | 6 | 7 | Total | noch im Magen festgeheftet | %-Wirksamkeit |
|---|---|---|---|---|---|---|---|---|---|---|
| Verbindung A | | | | | | | | | | |
| G. intestinalis | 2 | 16 | 18 | 5 | 0 | 0 | 0 | 41 | 3 | 93 |
| G.nasalis | 1 | 4 | 1 | 0 | 0 | 0 | 0 | 6 | 0 | 100 |
| Verbindung C | | | | | | | | | | |
| G. intestinalis | 1 | 2 | 8 | 10 | 7 | 2 | 0 | 30 | 0 | 100 |
| G. nasalis | 0 | 3 | 7 | 1 | 0 | 0 | 0 | 11 | 0 | 100 |

PATENTANSPRÜCHE:

1. Mittel gegen Magendasseln, gekennzeichnet durch einen Gehalt an einem Cyclopropancarbonsäureester der Formel I,

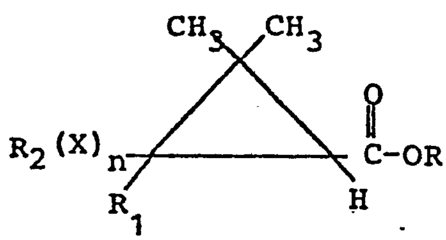

wobei

$R_1$ Wasserstoff oder Methyl,

$R_2$ $(C_1-C_4)$-Alkyl, $(C_1-C_4)$-Halogenalkyl mit 1-4 Halogenatomen wie Fluor, Chlor oder Brom, $(C_2-C_4)$-Alkenyl, $(C_2-C_4)$-Halogenalkenyl mit 1-4 Halogenatomen wie Fluor, Chlor oder Brom, Phenyl, Halogenphenyl mit 1-3 Halogenatomen wie Fluor oder Chlor,

X Sauerstoff oder Schwefel und

n O oder 1 bedeuten, mit der Maßgabe daß, wenn n 0 bedeutet,

$R_1$ und $R_2$ zusammen mit dem C-Atom, an das sie gebunden sind, eine cyclische Gruppe der Formel II oder III bedeuten,

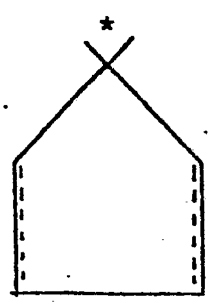

II

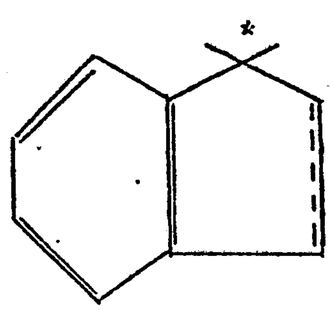

III

worin das mit einem (*) gekennzeichnete Kohlenstoffatom das mit dem Cyclopropanring gemeinsame Kohlenstoffatom ist, während ------- eine Einfach- oder Doppelbindung darstellt,

und wobei in der allgemeinen Formel I R einen Rest der Formel IV, V oder VI bedeutet,

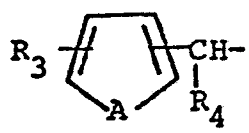 ; 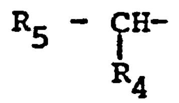 ; 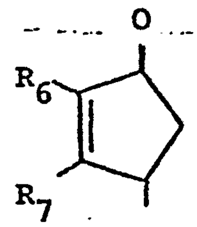

IV V VI

in welchen

$R_4$ Wasserstoff, Äthinyl oder Cyan,

$R_3$ Allyl, Propargyl, Benzyl, Thenyl, Furylmethyl, Phenoxy oder Phenylmercapto, welche Reste gegebenenfalls durch ein oder zwei Fluor- oder Chloratome oder durch Alkyl oder Alkoxy mit jeweils 1 bis 3 C-Atomen oder durch Tifluormethyl substituiert sein können,

A Sauerstoff, Schwefel oder -CH=CH-,

$R_5$ einen Phthalimid-, Thiophthalimid-, Di- oder Tetrahydrophthalimid- oder Dialkylmaleinimidrest,

$R_6$ $(C_1-C_3)$-Alkyl, $(C_2-C_4)$-Alkenyl, $(C_2-C_4)$-Alkinyl oder Benzyl und

$R_7$ Wasserstoff oder Methyl bedeuten,

in Mischung mit einem pharmazeutisch üblichen Trägerstoff und/oder Konstituenz.

2. Mittel gegen Magendasseln nach Anspruch 1, gekennzeichnet durch einen Gehalt an einem Cyclopropancarbonsäureester der Formel I in Anspruch 1, wobei

$R_1$ Wasserstoff,

$R_2$ Halogenäthyl mit 4-Halogenatomen aus der Gruppe Chlor und Brom, ω-Dichlorvinyl oder ω-Dibromvinyl,

n Null und

R den Rest

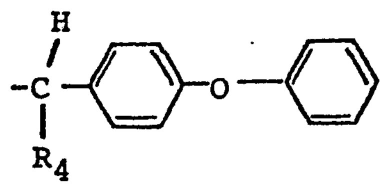

in welchem $R_4$ für Wasserstoff oder die Cyanogruppe steht,

bedeuten.

3. Mittel gegen Magendasseln nach Anspruch 1, gekennzeichnet durch einen Gehalt an (S)-α-Cyan-3-phenoxybenzyl(1R)-cis-3-(2,2-dibromvinyl)-2,2-dimethylcyclopropancarboxylat.

4. Mittel gegen Magendasseln nach Anspruch 1, gekennzeichnet durch einen Gehalt an (S)-α-Cyan-3-phenoxybenzyl(1R)-cis-3-(2,2-dichlorvinyl)-2,2-dimethylcyclopropancarboxylat.

5. Mittel gegen Magendasseln nach Anspruch 1, gekennzeichnet durch einen Gehalt an (S)-α-Cyan-3-phenoxybenzyl(1R)-cis-3-(2',2'-dichlor-1',2'-dibromäthyl)-2,2-dimethylcyclopropancarboxylat.

6. Mittel gegen Magendasseln nach Anspruch 1, gekennzeichnet durch einen Gehalt an α-Cyan-3-phenoxybenzyl-3-(2,2-dichlorvinyl)-2,2-dimethylcyclopropancarboxylat.

7. Mittel gegen Magendasseln nach Anspruch 1, gekennzeichnet durch einen Gehalt an 3-Phenoxybenzyl-3(2,2-dichlorvinyl)-2.2-dimethylcyclopropancarboxylat.

8. Verwendung eines Cyclopropancarbonsäureesters der Formel I in Anspruch 1 oder 2 zur Bekämpfung von Magendasseln.

9. Verwendung eines Cyclopropancarbonsäureesters der Formel I in Anspruch 1 oder 2 zur Herstellung eines Arzneimittels gegen Magendasseln.

0062704

Nummer der Anmeldung

EP 81 10 9868

Europäisches Patentamt

# EUROPÄISCHER RECHERCHENBERICHT

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | US - A - 4 078 080 (M.S.SCHRIDER)<br>* die ganze Patentschrift *<br>-- | 1-9 | A 61 K 31/275<br>A 61 K 31/215 |
| X | EP - A - 0 007 446 (CIBA-GEIGY)<br>* Ansprüche; Seiten 4 und 5 *<br>-- | 1-9 | |
| X | US - A - 4 225 533 (C.A.HENRICK)<br>* Ansprüche; Spalte 1 *<br>-- | 1-9 | RECHERCHIERTE SACHGEBIETE (Int. Cl.³) |
| X | CHEMICAL ABSTRACTS, Band 87, Nr. 15, 10. Oktober 1977 Zusammenfassung 113034d, Seiten 205,206 COLUMBUS, OHIO (US) & SOUTHWEST.ENTOMOL.1976 1(4)194-7 HARRIS R.L. u.a.: "Control of tabanids on horses"<br>* Zusammenfassung *<br>-- | 2,4,6, 7-9 | C 07 C 69/00<br>A 61 K 31/00 |
| D | CHEMICAL SOCIETY REVIEWS,Band 7, 1978 US M.ELLIOTT und N.F.JANES: "Synthetic Pyrethroids - A New Class of Insecticide" Seiten 5473-5505<br>* Artikel * | 1-9 | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| DEN HAAG | 27. April 1982 | BERTE |

EPA form 1503.1 06.78